# EUROPEAN PATENT SPECIFICATION

(11) **EP 4 431 138 B1**
(45) Date of publication and mention of the grant of the patent: **14.01.2026**
(21) Application number: 23168832.6
(22) Date of filing: 19.04.2023
(51) Int. Cl.: A61M 11/00, B05B 12/08, B05B 17/06, A61M 15/00, A61M 16/08, B05B 7/00, A61M 16/00

(54) **ATOMIZER AND ATOMIZING DEVICE**
ZERSTÄUBER UND ZERSTÄUBUNGSVORRICHTUNG
ATOMISEUR ET DISPOSITIF D'ATOMISATION

(30) Priority: 15.03.2023 CN 202320573426 U
(43) Date of publication of application: 18.09.2024
(73) Proprietor: Feellife Health Inc., Shenzhen, Guangdong 518000 (CN)
(72) Inventor: HUA, Jian, Shenzhen, Guangdong 518000 (CN); LIU, Lisi, Shenzhen, Guangdong 518000 (CN); LU, Jialin, Shenzhen, Guangdong 518000 (CN)
(74) Representative: Redl, Gerda

(56) References cited:
- WO-A1-2009/118717
- WO-A1-2016/151029
- US-A1- 2020 390 984
- US-A1- 2022 226 598
- US-A1- 2023 001 107

## Description

### TECHNICAL FIELD

The disclosure relates to the technical field of atomization, and particularly to an atomizer and an atomizing device.

### BACKGROUND

In the related art, there is a certain angle between a cup body and a cup mouth of an atomizing cup, so that the cup body avoids a power supply interface of the atomizer. However, due to the included angle between the cup body and the cup mouth, a corner may be formed inside the atomizing cup. During use, when the corner is located above an atomizing sheet, bubbles generated during atomization of the atomizing sheet may float to the corner, and when the formed bubbles are large, the bubbles may separate the atomizing sheet from the atomizing liquid, resulting in atomization interruption.

The document US20230001107A1 discloses that a nebulizer has an aerosol generator mounted in a housing, and has a vibratable aperture plate mounted to an annular support and to which is attached a vibration generator piezo. A downstream annular resilient seal is mounted between the housing and the washer on a side of the aperture plate opposed to the liquid supply reservoir. An upstream resilient seal is in the form of a gasket and is mounted between the washer and the housing reservoir, and has an opening forming part of a throat over the aperture plate. The gasket has a washer-shaped body extending radially from a downstream-extending rim adjacent the aperture plate. There are two upwardly-directed ridges for engagement with a housing surface, and the gasket is configured so that when under axial compression the opening internal surface is tapered inwardly in a flow direction to form a funnel shape.

The document US20200390984A1 discloses that a nebulization device having dual modules, which includes: a nebulization host machine; a three-way tube communicated with the nebulization host machine; a controller connected to the nebulization host machine; and a ventilator and a mask communicated with the three-way tube respectively; the nebulization host machine includes a side cover, a nozzle arranged on one side of the side cover, a liquid medicine bottle arranged on the other side of the side cover and communicated with the nozzle, a nebulization sheet arranged on one side of the liquid medicine bottle and communicated with the nozzle, and a first plug-in interface arranged below the liquid medicine bottle; a bottom portion in the nozzle is provided with a partition plate that divides an interior of the nozzle into an upper nebulization chamber and a lower gas flow chamber.

The document WO2009118717A1 discloses that apparatus used in insufflation comprises an insufflator for generating an insufflation gas such as carbon dioxide and an aerosol generator for aerosolising a fluid and entraining the aerosol with the insufflation gas which is delivered during surgery. The aerosol generator comprises a vibratable member having a plurality of apertures extending between a first surface and a second surface. The fluid may comprise a therapeutic or prophylactic agent. A controller is used to control the operation of the aerosol generator. The controller controls operation, of the aerosol generator responsive to the flow of insufflation' gas such as detected by a flow sensor. The pulse rate at a set frequency of vibration of the vibratable member is controlled.

The document US20220226598A1 discloses devices, systems, and methods for introducing a nebulized medication into breathing gas for respiratory therapy. An apparatus includes a reservoir to contain a volume of medication, a vibrating mesh disposed at a reservoir outlet of the reservoir to receive a flow of the medication from the reservoir and output a flow of nebulized medication, a mixing chamber having a gas inlet to receive a flow of breathing gas from a gas source, and a nebulizer inlet to receive the flow of nebulized medication from the vibrating mesh, where the vibrating mesh is disposed between the nebulizer inlet and the reservoir outlet.

The document WO2016151029A1 discloses that a liquid reservoir of a nebulizer has a funnel portion with ribs extending downwardly along a side wall towards a reservoir outlet. The ribs prevent the formation of air bubbles between the aperture plate and medication as any trapped air will be expelled along the geometry formed by the rib or groove and reservoir wall. Bubble formation is therefore prevented with only modification of the wall internal surface configuration of the reservoir.

### SUMMARY

The disclosure aims to solve at least one of the technical problems in the existing technology. Therefore, an atomizer provided by the disclosure can avoid bubbles from blocking a flow channel, and the bubbles are placed to separate an atomizing sheet from an atomizing liquid, thus preventing atomization interruption.

An atomizer according to an embodiment in a first aspect of the disclosure comprises:
an atomizing assembly comprising an atomizing sheet and configured for atomizing an atomizing liquid; and
an atomizing cup comprising a cup body, a power supply interface and a cup mouth, wherein the power supply interface is configured for being connected with an external electrical connector, the cup body and the power supply interface are located on the same side of the cup mouth, the cup body comprises a liquid storage portion, the liquid storage portion is provided with a liquid storage cavity, the liquid storage cavity is configured for storing the atomizing liquid, the atomizing cup is further provided with an atomizing cavity, the atomizing assembly is arranged in the atomizing cavity, the cup mouth is provided with a gas outlet, and the gas outlet is communicated with the atomizing cavity and configured for discharging mist generated in the atomizing cavity;
wherein the cup body further comprises a connecting portion, one side of the connecting portion is connected to the liquid storage portion, and the other side of the connecting portion is connected to the cup mouth, so as to define the atomizing cavity with the cup mouth; and
wherein a flow channel portion is further arranged between the atomizing assembly and the liquid storage portion, the flow channel portion is provided with a flow channel communicating the atomizing cavity with the liquid storage cavity, the liquid storage portion and the power supply interface are distributed along a radial direction of the flow channel, and the liquid storage portion is obliquely arranged away from the power supply interface, so as to avoid the power supply interface;
wherein the atomizing assembly further comprises an adapter sheet, the adapter sheet is provided with a through hole, the through hole is communicated with the flow channel, the atomizing sheet is located on one side of the adapter sheet away from the power supply interface and electrically connected with the adapter sheet, and the adapter sheet is configured for being electrically connected with the external electrical connector;
wherein, along the radial direction of the flow channel, a corner is formed on a side wall of the flow channel away from the power supply interface, a length between the corner and the adapter sheet is L, L≤3 mm, a diameter of the flow channel is D, D≥7 mm, and a part of the liquid storage cavity is located on one side of the corner away from the power supply interface ;
the atomizer further comprises a waterproof member, the waterproof member is arranged in the atomizing cavity, at least a part of the waterproof member is clamped between the connecting portion and the atomizing assembly, and the waterproof member and the connecting portion are jointly configured to form the flow channel portion;
wherein the waterproof member has an elastic structure, the waterproof member is provided with a mounting hole, a hole wall of the mounting hole is provided with a clamping groove, and an outer edge of the atomizing assembly is clamped into the clamping groove;
wherein the clamping groove is recessed along a radial direction of the mounting hole, and extends along a circumferential direction of the mounting hole, so as to form a closed annular groove.

The atomizer according to the embodiment of the disclosure at least has the following beneficial effects.

In the embodiment of the disclosure, because a part of the liquid storage cavity is located on one side of the corner away from the power supply interface, when the flow channel is in a horizontal position and the corner is located above the atomizing sheet, the part of the liquid storage cavity is higher than the corner. When the bubbles float to the corner, as the bubbles grow to a certain extent, the bubbles may break away from the corner and float into the liquid storage cavity. In addition, in the embodiment of disclosure, because the length L of the corner is less than or equal to 3 mm, and the diameter of the flow channel is greater than or equal to 7 mm, when the flow channel has not been completely covered by the bubbles, the bubbles will float into the liquid storage cavity, so as to avoid the bubbles from blocking the flow channel, thus preventing the bubbles from separating the atomizing sheet from the atomizing liquid, and preventing atomization interruption.

According to some embodiments of the disclosure, the adapter sheet is configured for abutting against the external connector, and the atomizing sheet is detachably connected with the adapter sheet.

According to some embodiments of the disclosure, along the radial direction of the flow channel, a distance between a center of the through hole and the corner is greater than a radius of the flow channel.

According to some embodiments of the disclosure, the cup mouth is further provided with a groove, the groove is provided with an opening, the cup body is connected to the opening, so as to define the liquid storage cavity, and the atomizing assembly is clamped between the cup mouth and the cup body.

According to some embodiments of the disclosure, the atomizing cup further comprises an expansion interface, the expansion interface is connected to the cup body, the expansion interface is provided with a liquid filling hole, the liquid filling hole is communicated with the liquid storage cavity, and the expansion interface is configured for being connected with an external liquid storage container.

An atomizing device according to an embodiment in a second aspect of the disclosure comprises the atomizer according to the embodiment in the first aspect.

The atomizing device according to the embodiment of the disclosure at least has the following beneficial effects.

The atomizer according to the embodiment in the first aspect is used, and in the atomizer according to the embodiment in the first aspect, because a part of the liquid storage cavity is located on one side of the corner away from the power supply interface in the embodiment, when the flow channel is in a horizontal position and the corner is located above the atomizing sheet, the part of the liquid storage cavity is higher than the corner. When the bubbles float to the corner, as the bubbles grow to a certain extent, the bubbles may break away from the corner and float into the liquid storage cavity. In addition, in the embodiment of the disclosure, because the length L of the corner is less than or equal to 3 mm, and the diameter of the flow channel is greater than or equal to 7 mm, when the flow channel has not been completely covered by the bubbles, the bubbles will float into the liquid storage cavity, so as to avoid the bubbles from blocking the flow channel, thus preventing the bubbles from separating the atomizing sheet from the atomizing liquid, and preventing atomization interruption.

According to some embodiments of the disclosure, the atomizing device further comprises a host, a data line and a three-way pipe;
the three-way pipe comprises a first interface, a second interface and a third interface, the atomizer is connected to the first interface, the second interface is configured for discharging mist for a user to use, and the third interface is configured for allowing gas to enter the three-way pipe; and
the data line comprises an input end and at least one output end, the input end is connected to the host, and the at least one output end is connected to the atomizer.

According to some embodiments of the disclosure, the atomizing device further comprises a gas channel assembly, the data line comprises two output ends, one of the output ends is connected to the atomizer and the other of the output ends is connected to the gas channel assembly, the gas channel assembly comprises a pipe body and a flow sensor, the flow sensor is arranged in the pipe body and configured for detecting a gas flow, and one end of the pipe body is communicated with the third interface and the other end of the pipe body is configured for allowing gas to enter the pipe body.

According to some embodiments of the disclosure, an included angle between an axis of the first interface and an axis of the second interface is α, and 90°<α<180°.

The additional aspects and advantages of the disclosure will be partially provided in the following description, and will partially be apparent in the following description, or learned by practice of the disclosure.

The invention is set out in the appended set of claims.

### BRIEF DESCRIPTION OF THE DRAWINGS

The disclosure is further described hereinafter with reference to the drawings and the embodiments, wherein:
FIG. 1 is a schematic structural diagram of an atomizer according to an embodiment of the disclosure;
FIG. 2 is a sectional view of FIG. 1;
FIG. 3 is an enlarged diagram of a region A in FIG. 2;
FIG. 4 is a schematic diagram marking dimension parameters of a flow channel and a through hole;
FIG. 5 is a sectional view of an atomizer according to another embodiment of the disclosure not encompassed by the wording of the claims;
FIG. 6 is an enlarged diagram of a region B in FIG. 5;
FIG. 7 is a schematic structural diagram of an atomizer according to another embodiment of the disclosure;
FIG. 8 is a schematic structural diagram of an atomizing device according to another embodiment of the disclosure; and
FIG. 9 is a schematic structural diagram of a three-way pipe in FIG. 8.

### Reference numerals:

1000 refers to atomizer;
100 refers to atomizing assembly, 110 refers to atomizing sheet, 120 refers to adapter sheet, and 121 refers to through hole;
200 refers to atomizing cup, 210 refers to cup body, 211 refers to liquid storage portion, 2111 refers to liquid storage cavity, 212 refers to connecting portion, 220 refers to cup mouth, 221 gas outlet, 230 refers to power supply interface, 240 refers to atomizing cavity, 250 refers to cup cover, and 260 refers to expansion interface;
300 refers to flow channel portion, 310 refers to flow channel, and 320 refers to corner;
400 refers to waterproof member, and 500 refers to host;
600 refers to data line, 610 refers to input end, and 620 refers to output end;
700 refers to three-way pipe, 710 refers to first interface, 720 refers to second interface, and 730 refers to third interface; and
800 refers to gas channel assembly, and 810 refers to pipe body.

### DETAILED DESCRIPTION

Embodiments of the disclosure are described in detail hereinafter, and examples of the embodiments are shown in the drawings, wherein the same or similar reference numerals throughout the drawings denote the same or similar elements or elements having the same or similar functions. The embodiments described hereinafter with reference to the drawings are exemplary, and are only used to explain the disclosure, but should not be understood as limiting the disclosure.

In the description of the disclosure, it should be understood that the orientations or positional relationships indicated by the terms such as "upper", "lower", "front", "rear", "left", "right" and the like, refer to the orientations or positional relationships shown in the drawings, which are only intended to facilitate describing the disclosure and simplifying the description, and do not indicate or imply that the indicated devices or elements must have a specific orientation, be constructed and operated in a specific orientation, and therefore cannot be understood as limiting the disclosure.

In the description of the disclosure, the meaning of several refers to be one or more, and the meaning of "a plurality of" refers to be two or more. The meanings of greater than, less than, more than, and the like are understood as not including the following number, while the meanings of above, below, within, and the like are understood as including the following number. If there is the description of first and second, it is only for the purpose of distinguishing between technical features, and should not be understood as indicating or implying relative importance, implicitly indicating the number of the indicated technical features or implicitly indicating the order of the indicated technical features.

In the description of the disclosure, the terms "arrangement", "installation", "connection", and the like should be understood in a broad sense unless otherwise specified and defined. The specific meaning of the above terms in the disclosure may be reasonably determined according to specific contents of the technical solutions by those of ordinary skills in the art.

In the description of the disclosure, the descriptions with reference to the terms "one embodiment", "some embodiments", "schematic embodiments", "examples", "specific examples", or "some examples" refer to that the specific features, structures, materials, or characteristics described in combination with the embodiment or example are included in at least one embodiment or example of the disclosure. In the specification, the schematic representation of the above terms does not necessarily mean the same embodiment or example.

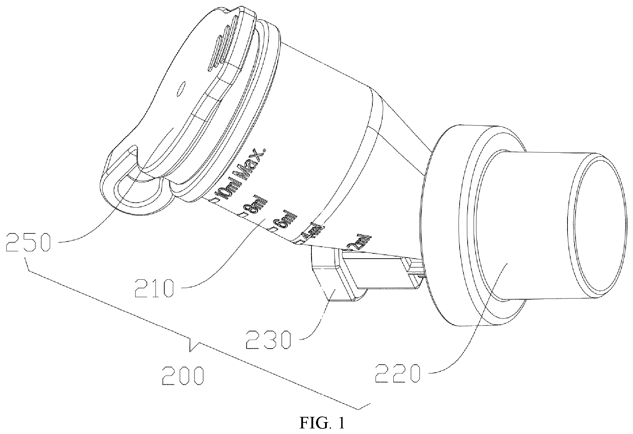

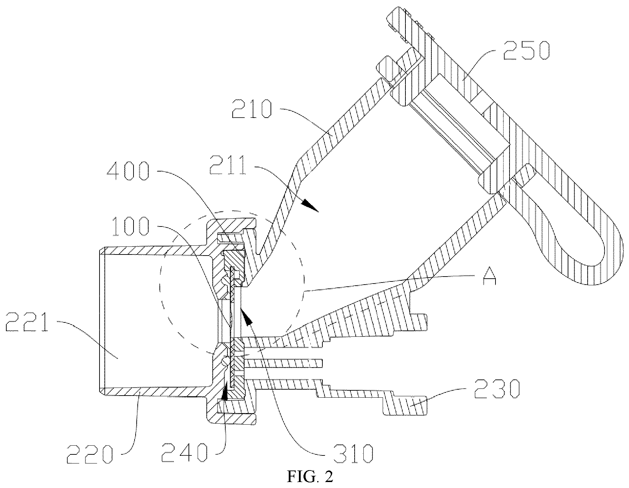

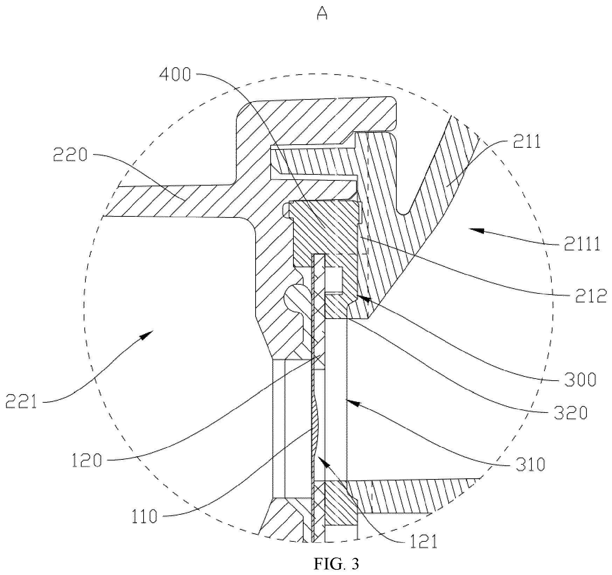

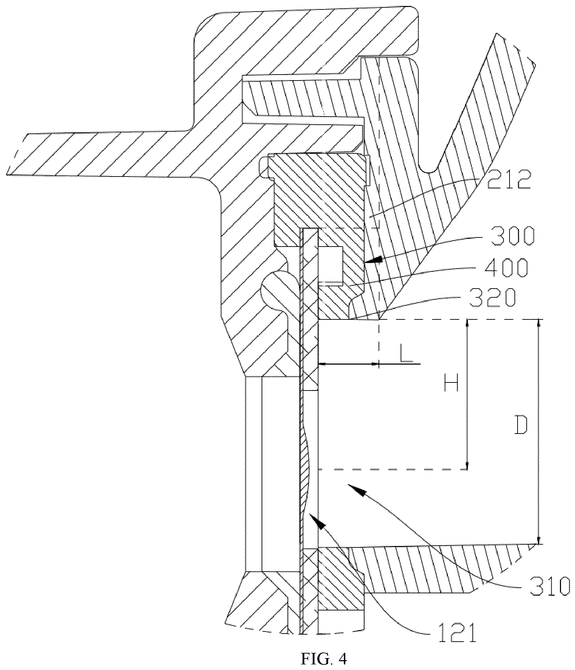

FIG. 1 is a schematic structural diagram of an atomizer according to an embodiment of the disclosure, FIG. 2 is a sectional view of FIG. 1, FIG. 3 is an enlarged diagram of a region A in FIG. 2, and FIG. 4 is a schematic diagram marking dimension parameters of a flow channel and a through hole. With reference to FIG. 1 to FIG. 4, an atomizer 1000 according to an embodiment in a first aspect comprises an atomizing assembly 100 and an atomizing cup 200.

The atomizing assembly 100 comprises an atomizing sheet 110 for atomizing an atomizing liquid. The atomizing cup 200 comprises a cup body 210, a cup mouth 220 and a power supply interface 230. The power supply interface 230 is configured for being connected with an external electrical connector, the cup body 210 and the power supply interface 230 are located on the same side of the cup mouth 220. The cup body 210 comprises a liquid storage portion 211, the liquid storage portion 211 is provided with a liquid storage cavity 2111, and the liquid storage cavity 2111 is configured for storing the atomizing liquid. The cup body 210 may be made of a transparent material, or partially made of a transparent material, so that a user can visually observe the atomizing liquid in the liquid storage cavity 2111, and the user can replace the atomizing liquid in time, thus preventing the atomizing sheet 110 from dry burning. The atomizing cup 200 is further provided with an atomizing cavity 240, the atomizing assembly 100 is arranged in the atomizing cavity 240, the cup mouth 220 is provided with a gas outlet 221, and the gas outlet 221 is communicated with the atomizing cavity 240 and configured for discharging mist generated in the atomizing cavity 240.

A flow channel portion 300 is further arranged between the atomizing assembly 100 and the liquid storage portion 211, the flow channel portion 300 is provided with a flow channel 310, the flow channel 310 communicates the atomizing cavity 240 with the liquid storage cavity 2111, the liquid storage portion 211 and the power supply interface 230 are distributed along a radial direction of the flow channel 310, and the liquid storage portion 211 is obliquely arranged away from the power supply interface 230, so as to avoid the power supply interface 230. For example, an included angle between the cup body 210 and the cup mouth may 135°, 150°, and the like, which is set according to sizes of the cup body 210 and the power supply interface 230, so as to form a mounting space for the power supply interface 230. Along the radial direction of the flow channel 310, a corner 320 is formed on a side wall of the flow channel 310 away from the power supply interface 230, a length of the corner 320 is L, L≤3 mm, a diameter of the flow channel is D, D≥7mm, and a part of the liquid storage cavity 2111 is located on one side of the corner 320 away from the power supply interface 230.

A part of the liquid storage cavity 2111 is located on one side of the corner 320 away from the power supply interface 230. Therefore, during use, when the flow channel 310 is in a horizontal position, and the corner 320 is located above the atomizing sheet 110, the part of the liquid storage cavity 2111 is higher than the corner 320. When bubbles float to the corner 320, as the bubbles grow to a certain extent, the bubbles may break away from the corner 320 and float into the liquid storage cavity 2111 from the flow channel 310.

In addition, the longer the length of the corner 320 is, the stronger the capability of restraining the bubbles will be, and the smaller the diameter of the flow channel 310 is, the easier the flow channel will be blocked by the bubbles. On this basis, experiments were carried out with multiple groups of different lengths L of the corner 320 and different diameters D of the flow channel 310, and the interruption of the atomizer 1000 is verified through the experiments. It is learned through the experiments that, when the length L of the corner 320 is less than or equal to 3 mm and the diameter D of the flow channel 310 is greater than or equal to 7 mm, the bubbles may float into the liquid storage cavity 2111 when the bubbles grow to an extent of not completely covering the flow channel 310, so as to avoid the bubbles from blocking the flow channel 310, thus preventing the bubbles from separating the atomizing sheet 110 from the atomizing liquid and preventing atomization interruption.

During the experiments, multiple groups of experiments were set (only four groups are provided below), and each group of experiment is provided with multiple data (only seven data are provided below). In order to reduce experimental errors, at least 10 atomizers 1000 were set for each data, and each atomizer 1000 ran for 30 minutes, so as to detect whether or not the atomizer 1000 would be interrupted. Experimental results are shown in the following tables:

### First group of experiment:

| Serial number | 1 | 2 | 3 | 4 | 5 | 6 | 7 |
|---|---|---|---|---|---|---|---|
| L (mm) | 1.5 | 1.5 | 1.5 | 1.5 | 1.5 | 1.5 | 1.5 |
| D (mm) | 4 | 5 | 6 | 7 | 8 | 9 | 10 |
| Whether or not the atomizer was interrupted | Yes | Yes | Yes | No | No | No | No |

### Second group of experiment:

| Serial number | 1 | 2 | 3 | 4 | 5 | 6 | 7 |
|---|---|---|---|---|---|---|---|
| L (mm) | 2 | 2 | 2 | 2 | 2 | 2 | 2 |
| D (mm) | 4 | 5 | 6 | 7 | 8 | 9 | 10 |
| Whether or not the atomizer was interrupted | Yes | Yes | Yes | No | No | No | No |

### Third group of experiment:

| Serial number | 1 | 2 | 3 | 4 | 5 | 6 | 7 |
|---|---|---|---|---|---|---|---|
| L (mm) | 3 | 3 | 3 | 3 | 3 | 3 | 3 |
| D (mm) | 4 | 5 | 6 | 7 | 8 | 9 | 10 |
| Whether or not the atomizer was interrupted | Yes | Yes | Yes | No | No | No | No |

### Fourth group of experiment:

| Serial number | 1 | 2 | 3 | 4 | 5 | 6 | 7 |
|---|---|---|---|---|---|---|---|
| L (mm) | 4 | 4 | 4 | 4 | 4 | 4 | 4 |
| D (mm) | 4 | 5 | 6 | 7 | 8 | 9 | 10 |
| Whether or not the atomizer was interrupted | Yes | Yes | Yes | Yes | Yes | Yes | Yes |

It is learn from the first group of experiment that, in the case that the length of the corner 320 is 1.5 mm, when the diameter of the flow channel 310 is greater than or equal to 7 mm, the atomizer 1000 will not be interrupted.

It is learn from the second group of experiment that, in the case that the length of the corner 320 is 2 mm, when the diameter of the flow channel 310 is greater than or equal to 7 mm, the atomizer 1000 will not be interrupted.

It is learn from the third group of experiment that, in the case that the length of the corner 320 is 3 mm, when the diameter of the flow channel 310 is greater than or equal to 7 mm, the atomizer 1000 will not be interrupted.

It is learn from the fourth group of experiment that, in the case that the length of the corner 320 is 4 mm, the atomizer 1000 will be interrupted. Thus, it can be seen that, when the length of the corner 320 is less than or equal to 3 mm, and the diameter of the flow channel 310 is greater than or equal to 7 mm, the bubbles may not separate the atomizing sheet 110 from the atomizing liquid, so that the atomizer 1000 will not be interrupted during working.

With reference to FIG. 2, FIG. 3, FIG. 5 and FIG. 6, FIG. 5 is a sectional view of an atomizer not encompassed by the wording of the claims.

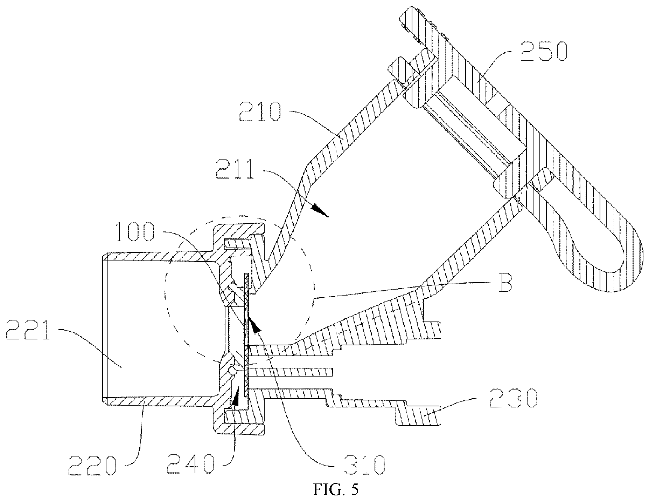

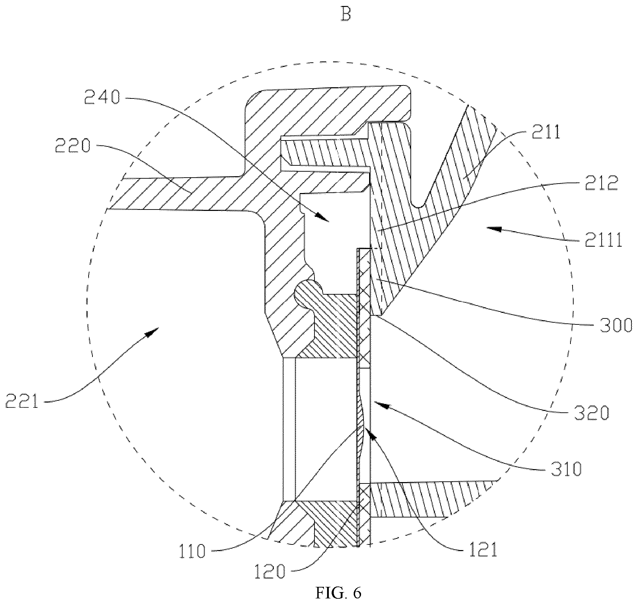

FIG. 6 is an enlarged diagram of a region B in FIG. 5. The cup body 210 further comprises a connecting portion 212, one side of the connecting portion 212 is connected to the liquid storage portion 211, and the other side of the connecting portion 212 is connected to the cup mouth, so as to define the atomizing cavity 240 with the cup mouth 220. The atomizing assembly 100 abuts against one side of the connecting portion 212 away from the liquid storage portion 211, and the connecting portion 212 is configured to form the flow channel portion 300 (as shown in FIG. 6). The atomizer 1000 further comprises a waterproof member 400, and the waterproof member 400 may be a prefabricated part or a waterproof glue. The waterproof member 400 is arranged in the atomizing cavity 240, at least a part of the waterproof member 400 is clamped between the connecting portion 212 and the atomizing assembly 100, and the waterproof member 400 and the connecting portion 212 are configured to jointly form the flow channel portion 300 (as shown in FIG. 4).

The waterproof member 400 is configured for improving a sealing performance between the connecting portion 212 and the atomizing assembly 100, thus preventing a gap from being formed between the atomizing assembly 100 and a cavity wall of the atomizing cavity 240 to make the atomizing liquid not subjected to atomization flow into the atomizing cavity 240. On one hand, the atomizing liquid can be prevented from leaking when the atomizer 1000 is not in use. On the other hand, the atomizing liquid not subjected to atomization is prevented from being directly inhaled into human body when the atomizer is in use.

With reference to FIG. 3, the waterproof member 400 has an elastic structure, the waterproof member 400 is provided with a mounting hole, a hole wall of the mounting hole is provided with a clamping groove, the atomizing assembly 100 is arranged in the mounting hole, and an outer edge of the atomizing assembly 100 is clamped into the clamping groove. The clamping groove is recessed along a radial direction of the mounting hole, and extends along a circumferential direction of the mounting hole, so as to form a closed annular groove. During mounting, the atomizing assembly 100 is inserted into the mounting hole first, so that the waterproof member 400 is pressed and deformed. When the edge of the atomizing assembly 100 enters the clamping groove, the waterproof member 400 is restored under an elastic effect. At the moment, an inner wall of the waterproof member 400 abuts against the outer edge of the atomizing assembly 100, and an outer wall of the waterproof member 400 is attached to the inner wall of the atomizing cavity 240, thus improving a sealing performance between the atomizing assembly 100 and the atomizing cup 200.

With reference to FIG. 2 and FIG. 3, the atomizing assembly 100 further comprises an adapter sheet 120, the adapter sheet 120 is configured for being electrically connected with an external electrical connector, and the adapter sheet 120 is electrically connected with the atomizing sheet 110. The adapter sheet 120 is provided with a through hole 121, and the atomizing sheet 110 is connected to one side of the adapter sheet 120 away from the interface and electrically connected with the adapter sheet 120, so as to supply power to the atomizing sheet 110. The through hole 121 is communicated with the flow channel 310 and configured for exposing the atomizing sheet 110, so that the atomizing sheet 110 is contacted with the atomizing liquid in the flow channel 310. Specifically, the atomizing sheet 110 is electrically connected with the adapter sheet 120. Therefore, during manufacturing, it is only necessary to design the adapter sheet 120 in a corresponding shape to adapt to the existing atomizing sheet 110 in the market, without needing to design a structure of the atomizer 1000 according to a specification of the existing atomizing sheet 110, thus improving the design flexibility of the atomizer 1000 and simplifying the design difficulty.

Based on the above embodiment, the adapter sheet 120 is configured for abutting against the external electrical connector, and the adapter sheet 120 is detachably connected with the atomizing sheet 110. Specifically, for example, the external connector is a pogo pin. After the pogo pin is connected with the power supply interface 230, it extends into the atomizing cavity 240 and abuts against the adapter sheet 120, so as to supply power to the adapter sheet 120. It can be understood that, after using for many times, wear may occur between the pogo pin and the adapter sheet 120, so that replacement is required. Because the adapter sheet 120 and the atomizing sheet 110 are detachably connected in the embodiment of the disclosure, it is only necessary to disassemble the adapter sheet 120 during replacement, thus saving the maintenance cost.

With reference to FIG. 4, in some embodiments, along the radial direction of the flow channel 310, a distance H between a center of the through hole 121 and the corner 320 is greater than a radius R of the flow channel 310. That is, when the flow channel 310 is horizontally arranged and the corner 320 is located above the atomizing sheet 110, a center of the atomizing sheet 110 is located below a center of the flow channel 310, which means that the atomizing sheet 110 is located at a relatively low position of the flow channel 310, thus reducing the residual atomizing liquid. As shown in FIG. 4, the lowest edge of the atomizing sheet 110 is flush with the lowest edge of the flow channel 310, so that the atomizing liquid flowing into the flow channel 310 can all be contacted with the atomizing sheet 110. Therefore, the atomizing liquid in the flow channel 310 can be atomized by being contacted with the atomizing sheet 110, thus reducing waste of the atomizing liquid and saving a use cost of the user. In addition, for example, when the atomizing liquid is a drug, a drug effect may be reduced if the drug remains for a long time, or even unpredictable side effects may occur. In the embodiment of the disclosure, eccentric arrangement of the atomizing sheet 110 and the flow channel 310 can reduce the residual atomizing liquid, so as to ensure a therapeutic effect.

With reference to FIG. 2, in some embodiments, one end of the cup mouth 220 facing the cup body is provided with a groove, the groove is provided with an opening, the cup body 210 is inserted into the cup mouth 220 and covers the opening of the cup mouth, so as to form the atomizing cavity 240, and the atomizing assembly 100 is clamped between the cup mouth 220 and the cup body 210. Therefore, the atomizing sheet 110 is simpler to fix, and the atomizing assembly 100 is fixed without other connectors, thus simplifying an assembly process and improving assembly efficiency. Alternatively, in some embodiments, the waterproof member 400 is arranged at the opening, the mounting hole of the waterproof member 400 and the groove define the atomizing cavity 240, and the waterproof member 400 is clamped between the cup mouth 220 and the cup body 210, as shown in FIG. 2.

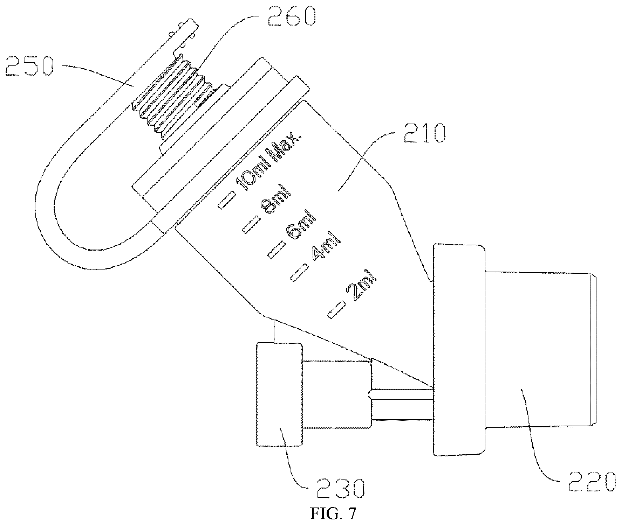

With reference to FIG. 7, FIG. 7 is a schematic structural diagram of an atomizer according to another embodiment of the disclosure. In some embodiments, the atomizing cup 200 further comprises an expansion interface 260, the expansion interface 260 is connected to the cup body 210, the expansion interface 260 is provided with a liquid filling hole, and the liquid filling hole is communicated with the liquid storage cavity 2111 and configured for adding the atomizing liquid to the liquid storage cavity 2111. The expansion interface 260 is configured for being connected with an external liquid storage container. On one hand, it facilitates connection of the external liquid storage container with the atomizing cup 200 when adding the atomizing liquid. On the other hand, for example, according to an actual demand, when a large amount of atomizing liquid needs to be used at a time, a liquid storage container meeting requirements may be directly connected to the expansion interface 260. Therefore, it is not necessary to add the atomizing liquid frequently during use, thus being more convenient to use. An outer wall of the expansion interface 260 is provided with a thread, so that the external liquid storage container is connected with the atomizer 1000 in the embodiment of the disclosure through the thread. In addition, the expansion interface 260 may be further provided with a magnetic suction member, a fastener, and the like, so that the external liquid storage container can be quickly connected with the atomizer 1000 in the embodiment of the disclosure.

In addition, the atomizer 1000 further comprises a cup cover 250, the cup cover 250 is detachably connected to the liquid filling hole, so as to selectively open or close the liquid filling hole, such as opening the cup cover 250 when adding the atomizing liquid, and blocking the liquid filling hole by the cup cover 250 after adding the atomizing liquid, thus preventing the drug liquid from being polluted and leaking.

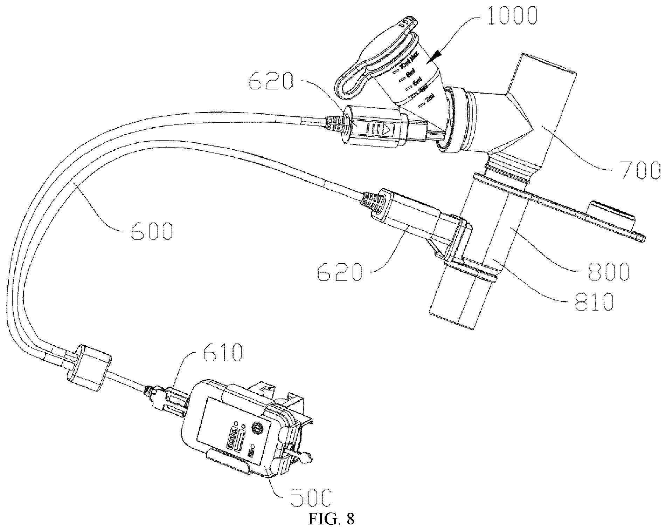

With reference to FIG. 8, FIG. 8 is a schematic structural diagram of an atomizing device according to another embodiment of the disclosure. An atomizing device according to an embodiment in a second aspect comprises the atomizer 1000 according to the embodiment in the first aspect. Because the atomizer 1000 according to the embodiment in the first aspect is used in the embodiment, and in the atomizer 1000 according to the embodiment in the first aspect, a part of the liquid storage cavity 2111 is located on one side of the corner 320 away from the power supply interface 230, when the flow channel 310 is in a horizontal position and the corner 320 is located above the atomizing sheet 110, the part of the liquid storage cavity is higher than the corner 320. When bubbles float to the corner 320, as the bubbles grow to a certain extent, the bubbles may break away from the corner 320 and float into the liquid storage cavity 2111. In addition, in the embodiment, because the length L of the corner 320 is less than or equal to 3 mm, and the diameter of the flow channel 310 is greater than or equal to 7 mm, when the flow channel 310 has not been completely covered by the bubbles, the bubbles will float into the liquid storage cavity 2111, so as to avoid the bubbles from blocking the flow channel 310, thus preventing the bubbles from separating the atomizing sheet 110 from the atomizing liquid, and preventing atomization interruption.

It should also be noted that, because all technical features of the atomizer 1000 according to the embodiment in the first aspect are used in this embodiment, this embodiment has all beneficial effects brought by the embodiment in the first aspect, which will not be repeated herein.

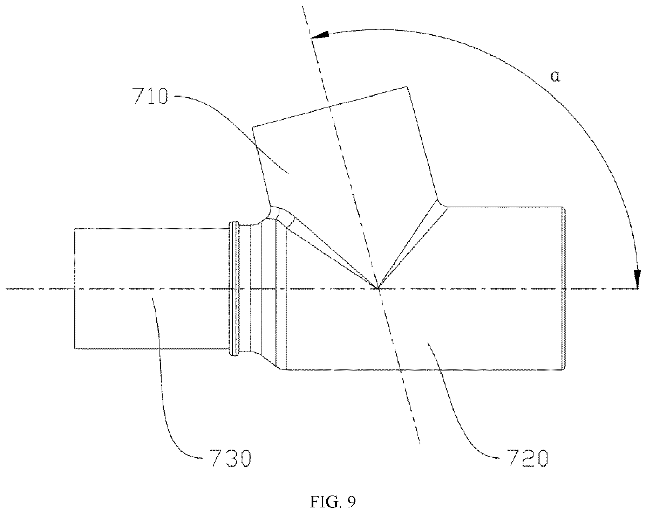

With reference to FIG. 8 and FIG. 9, FIG. 9 is a schematic structural diagram of a three-way pipe in FIG. 8. In some embodiments, the atomizing device further comprises a host 500, a data line 600 and a three-way pipe 700. The data line 600 comprises an input end 610 and at least one output end 620, the input end 610 is connected to the host 500, and the at least one output end 620 is connected to the atomizer 1000. The host 500 is configured for supplying power to the atomizer 1000, and may also be configured for controlling an atomizing function of the atomizer 1000, for example, controlling start or stop of the atomizer 1000, the atomizing speed, etc. The three-way pipe 700 comprises a first interface 710, a second interface 720 and a third interface 730. The atomizer 1000 is connected to the first interface 710, and the second interface 720 is configured for discharging mist for a user to use. The third interface 730 is configured for allowing gas to enter the three-way pipe 700. For example, the third interface 730 is directly communicated with an external space. During use by the user, air can enter the three-way pipe 700 through the third interface 730, and the mist enters the three-way pipe through the first interface 710. Therefore, the mist can flow to the user together with the air through the second interface 720, so that the user inhales the mist more smoothly, thus improving the user experience. In addition, when the atomizing device in this embodiment is used for treatment, the third interface 730 may also be configured for being communicated with an oxygen supply system to meet the oxygen supply requirements.

With reference to FIG. 8, based on the above embodiment, the atomizing device further comprises a gas channel assembly 800, the data line 600 comprises two output ends 620, and one output end 620 is connected to the atomizer and the other output end 620 is connected to the gas channel assembly 800. The gas channel assembly 800 comprises a pipe body 810 and a flow sensor, and the flow sensor is arranged in the pipe body 810 and configured for detecting a gas flow. One end of the pipe body 810 is communicated with the third interface 730 and the other end of the pipe body is configured for allowing gas to enter the pipe body. During use, the user inhales through the second interface 720 of the three-way pipe 700, and the gas enters the three-way pipe 700 through the third interface 730 to be contacted with the flow sensor. The flow sensor detects that a gas flow passes through and feeds back a detection signal to the host 500, and the host 500 controls the atomizing sheet 110 in the atomizer 1000 to start. When the user stops inhaling or exhaling, the flow sensor will give feedback to the host 500 so that the atomizing sheet 110 is switched to a closed state, thus avoiding the waste of the atomizing liquid.

With reference to FIG. 9, in some embodiments, an included angle between an axis of the first interface 710 and an axis of the second interface 720 is α, and 90°<α<180°. For example, when α is 105°, an included angle between a direction of the mist entering from the first interface 710 and a direction of the gas in the second interface 720 is 105°. Therefore, when the mist enters the second interface 720, the mist has a velocity component in the same direction as a flow direction of the gas in the second interface 720, so that the mist can quickly reach a flow velocity of the gas in the second interface 720 after entering the second interface 720. Therefore, the mist can quickly flow to the user through the second interface 720, so as to avoid more mist from being condensed due to contact with a pipe wall of the second interface 720 and being unable to be used effectively, thus reducing the waste of the atomizing liquid.

The embodiments of the disclosure are described in detail with reference to the drawings above, but the invention is not limited to the above embodiments, and various changes may also be made within the knowledge scope of those of ordinary skills in the art as defined by the appended claims.

## Claims

1. An atomizer (1000), comprising:
an atomizing assembly (100) comprising an atomizing sheet (110) and configured for atomizing an atomizing liquid; and
an atomizing cup (200) comprising a cup body (210), a power supply interface (230) and a cup mouth (220), wherein the power supply interface (230) is configured for being connected with an external electrical connector, the cup body (210) and the power supply interface (230) are located on the same side of the cup mouth (220), the cup body (210) comprises a liquid storage portion (211), the liquid storage portion (211) is provided with a liquid storage cavity (2111), the liquid storage cavity (2111) is configured for storing the atomizing liquid, the atomizing cup (200) is further provided with an atomizing cavity (240), the atomizing assembly (100) is arranged in the atomizing cavity (240), the cup mouth (220) is provided with a gas outlet (221), and the gas outlet (221) is communicated with the atomizing cavity (240) and configured for discharging mist generated in the atomizing cavity (240), wherein the cup body (210) further comprises a connecting portion (212), one side of the connecting portion (212) is connected to the liquid storage portion (211), and the other side of the connecting portion (212) is connected to the cup mouth (220), so as to define the atomizing cavity (240) with the cup mouth (220); and
wherein a flow channel portion (300) is further arranged between the atomizing assembly (100) and the liquid storage portion (211), the flow channel portion (300) is provided with a flow channel (310) communicating the atomizing cavity (240) with the liquid storage cavity (2111), the liquid storage portion (211) and the power supply interface (230) are distributed along a radial direction of the flow channel (310), and the liquid storage portion (211) is obliquely arranged away from the power supply interface (230), so as to avoid the power supply interface (230);
wherein the atomizing assembly (100) further comprises an adapter sheet (120), the adapter sheet (120) is provided with a through hole (121), the through hole (121) is communicated with the flow channel (310), the atomizing sheet (110) is located on one side of the adapter sheet (120) away from the power supply interface (230) and electrically connected with the adapter sheet (120), and the adapter sheet (120) is configured for being electrically connected with the external electrical connector;
wherein, along the radial direction of the flow channel (310), a corner (320) is formed on a side wall of the flow channel (310) away from the power supply interface (230), a length between the corner (320) and the adapter sheet (120) is L, L≤3 mm, a diameter of the flow channel (310) is D, D≥7mm, and a part of the liquid storage cavity (2111) is located on one side of the corner (320) away from the power supply interface (230);
wherein the atomizer (1000) further comprises a waterproof member (400), the waterproof member (400) is arranged in the atomizing cavity (240), at least a part of the waterproof member (400) is clamped between the connecting portion (212) and the atomizing assembly (100), and the waterproof member (400) and the connecting portion (212) are jointly configured to form the flow channel portion (300); wherein the waterproof member (400) has an elastic structure, the waterproof member (400) is provided with a mounting hole,
and **characterized in that** a hole wall of the mounting hole is provided with a clamping groove, and an outer edge of the atomizing assembly (100) is clamped into the clamping groove; wherein the clamping groove is recessed along a radial direction of the mounting hole, and extends along a circumferential direction of the mounting hole, so as to form a closed annular groove.

2. The atomizer (1000) according to claim 1, wherein the adapter sheet (120) is configured for abutting against the external connector, and the atomizing sheet (110) is detachably connected with the adapter sheet (120).

3. The atomizer (1000) according to claim 1, wherein along the radial direction of the flow channel (310), a distance between a center of the through hole (121) and the corner (320) is greater than a radius of the flow channel (310).

4. The atomizer (1000) according to claim 1, wherein the cup mouth (220) is further provided with a groove, the groove is provided with an opening, the cup body (210) is connected to the opening, so as to define the liquid storage cavity (2111), and the atomizing assembly (100) is clamped between the cup mouth (220) and the cup body (210).

5. The atomizer (1000) according to claim 1, wherein the atomizing cup (200) further comprises an expansion interface (260), the expansion interface (260) is connected to the cup body (210), the expansion interface (260) is provided with a liquid filling hole, the liquid filling hole is communicated with the liquid storage cavity (2111), and the expansion interface (260) is configured for being connected with an external liquid storage container.

6. An atomizing device, comprising the atomizer (1000) according to any one of claims 1 to 5.

7. The atomizing device according to claim 6, wherein the atomizing device further comprises a host (500), a data line (600) and a three-way pipe (700);
the three-way pipe (700) comprises a first interface (710), a second interface (720)and a third interface (730), the atomizer (1000) is connected to the first interface (710), the second interface (720)is configured for discharging mist for a user to use, and the third interface (730) is configured for allowing gas to enter the three-way pipe (700); and
the data line (600) comprises an input end (610) and at least one output end (620), the input end (610) is connected to the host (500), and the at least one output end (620) is connected to the atomizer (1000).

8. The atomizing device according to claim 7, wherein the atomizing device further comprises a gas channel assembly (800), the data line (600) comprises two output ends (620), one of the output ends (620) is connected to the atomizer (1000) and the other of the output ends (620) is connected to the gas channel assembly (800), the gas channel assembly (800) comprises a pipe body (810) and a flow sensor, the flow sensor is arranged in the pipe body (810) and configured for detecting a gas flow, and one end of the pipe body (810) is communicated with the third interface (730) and the other end of the pipe body (810) is configured for allowing gas to enter the pipe body (810).

9. The atomizing device according to claim 7 or 8, wherein an included angle between an axis of the first interface (710) and an axis of the second interface (720) is α, and 90°<α<180°.

## Patentansprüche

1. Zerstäuber (1000) umfassend:
eine Zerstäubungsanordnung (100), die eine Zerstäubungsfolie (110) umfasst und dazu ausgestaltet ist, eine Zerstäubungsflüssigkeit zu zerstäuben; und eine Zerstäubungsschale (200) umfassend einen Schalenkörper (210), eine Leistungsversorgungsschnittstelle (230) und eine Schalenöffnung (220), wobei die Leistungsversorgungsschnittstelle (230) dazu ausgestaltet ist, mit einem externen elektrischen Verbinder verbunden zu werden; der Schalenkörper (210) und die Leistungsversorgungsschnittstelle (230) sich auf derselben Seite der Schalenöffnung (220) befinden, der Schalenkörper (210) einen Flüssigkeitsspeicherabschnitt (211) umfasst, der Flüssigkeitsspeicherabschnitt (211) mit einer Flüssigkeitsspeicherkammer (2111) versehen ist, die Flüssigkeitsspeicherkammer (2111) dazu ausgestaltet ist, die Zerstäubungsflüssigkeit zu speichern, die Zerstäubungsschale (200) ferner mit einer Zerstäubungskammer (240) versehen ist, die Zerstäubungsanordnung (100) in der Zerstäubungskammer (240) angeordnet ist, die Schalenöffnung (220) mit einem Gasauslass (221) versehen ist und der Gasauslass (221) mit der Zerstäubungskammer (240) in Kommunikation steht und dazu ausgestaltet ist, einen Nebel abzugeben, der in der Zerstäubungskammer (240) erzeugt wird, wobei der Schalenkörper (210) ferner einen Verbindungsabschnitt (212) umfasst, wobei eine Seite des Verbindungsabschnitts (212) mit dem Flüssigkeitsspeicherabschnitt (211) verbunden ist und die andere Seite des Verbindungsabschnitts (212) mit der Schalenöffnung (220) verbunden ist, um die Zerstäubungskammer (240) mit der Schalenöffnung (220) zu definieren; und
wobei zwischen der Zerstäubungsanordnung (100) und dem Flüssigkeitsspeicherabschnitt (211) ferner ein Strömungskanalabschnitt (300) angeordnet ist, wobei der Strömungskanalabschnitt (300) mit einem Strömungskanal (310) versehen ist, der die Zerstäubungskammer (240) mit der Flüssigkeitsspeicherkammer (2111) in Kommunikation versetzt, der Flüssigkeitsspeicherabschnitt (211) und die Leistungsversorgungsschnittstelle (230) entlang einer radialen Richtung des Strömungskanals (310) verteilt sind, und der Flüssigkeitsspeicherabschnitt (211) schräg von der Leistungsversorgungsschnittstelle (230) entfernt angeordnet ist, um der Leistungsversorgungsschnittstelle (230) auszuweichen;
wobei die Zerstäubungsanordnung (100) ferner eine Adapterfolie (120) umfasst, die Adapterfolie (120) mit einem Durchgangsloch (121) versehen ist, das Durchgangsloch (121) mit dem Strömungskanal (310) in Kommunikation steht, die Zerstäubungsfolie (110) sich auf einer Seite der Adapterfolie (120) befindet, die von der Leistungsversorgungsschnittstelle (230) entfernt ist, und elektrisch mit der Adapterfolie (120) verbunden ist, und die Adapterfolie (120) dazu ausgestaltet ist, elektrisch mit dem externen elektrischen Verbinder verbunden zu werden;
wobei entlang der radialen Richtung des Strömungskanals (310) eine Ecke (320) an einer Seitenwand des Strömungskanals (310) von der Leistungsversorgungsschnittstelle (230) entfernt gebildet ist, wobei eine Länge zwischen der Ecke (320) und der Adapterfolie (120) L ist und L ≤ 3 mm gilt, ein Durchmesser des Strömungskanals (310) D ist und D ≥ 7mm gilt, und ein Teil der Flüssigkeitsspeicherkammer (2111) sich auf einer Seite der Ecke (320) befindet, die von der Leistungsversorgungsschnittstelle (230) entfernt ist;
wobei der Zerstäuber (1000) ferner ein wasserfestes Element (400) umfasst, das wasserfeste Element (400) in der Zerstäubungskammer (240) angeordnet ist, zumindest ein Teil des wasserfesten Elements (400) zwischen dem Verbindungsabschnitt (212) und der Zerstäubungsanordnung (100) eingeklemmt ist und das wasserfeste Element (400) und der Verbindungsabschnitt (212) gemeinsam dazu ausgestaltet sind, den Strömungskanalabschnitt (300) zu bilden; wobei das wasserfeste Element (400) eine elastische Struktur aufweist, wobei das wasserfeste Element (400) mit einem Montageloch versehen ist, und **dadurch gekennzeichnet, dass** eine Lochwand des Montagelochs mit einer Klemmnut versehen ist, und ein äußerer Rand der Zerstäubungsanordnung (100) in die Klemmnut geklemmt ist; wobei die Klemmnut entlang einer radialen Richtung des Montagelochs vertieft ist und sich entlang einer Umfangsrichtung des Montagelochs erstreckt, um so eine geschlossene ringförmige Nut zu bilden.

2. Zerstäuber (1000) nach Anspruch 1, wobei die Adapterfolie (120) dazu ausgestaltet ist, gegen den externen Verbinder anzuliegen, und die Zerstäubungsfolie (110) abnehmbar mit der Adapterfolie (120) verbunden ist.

3. Zerstäuber (1000) nach Anspruch 1, wobei entlang der radialen Richtung des Strömungskanals (310) ein Abstand zwischen einer Mitte des Durchgangslochs (121) und der Ecke (320) größer als ein Radius des Strömungskanals (310) ist.

4. Zerstäuber (1000) nach Anspruch 1, wobei die Schalenöffnung (220) ferner mit einer Nut versehen ist, die Nut mit einer Öffnung versehen ist, der Schalenkörper (210) mit der Öffnung verbunden ist, um so die Flüssigkeitsspeicherkammer (2111) zu definieren, und die Zerstäubungsanordnung (100) zwischen der Schalenöffnung (220) und dem Schalenkörper (210) eingeklemmt ist.

5. Zerstäuber (1000) nach Anspruch 1, wobei die Zerstäubungsschale (200) ferner eine Erweiterungsschnittstelle (260) umfasst, die Erweiterungsschnittstelle (260) mit dem Schalenkörper (210) verbunden ist, die Erweiterungsschnittstelle (260) mit einem Flüssigkeits-Füllloch versehen ist, das Flüssigkeits-Füllloch mit der Flüssigkeitsspeicherkammer (2111) in Kommunikation steht und die Erweiterungsschnittstelle (260) dazu ausgestaltet ist, mit einem externen Flüssigkeitsspeicherbehälter verbunden zu werden.

6. Zerstäubungsvorrichtung, umfassend den Zerstäuber (1000) nach einem der Ansprüche 1 bis 5.

7. Zerstäubungsvorrichtung nach Anspruch 6, wobei die Zerstäubungsvorrichtung ferner einen Host (500), eine Datenleitung (600) und ein Dreiweg-Rohr (700) umfasst; wobei das Dreiweg-Rohr (700) eine erste Schnittstelle (710), eine zweite Schnittstelle (720) und eine dritte Schnittstelle (730) umfasst, der Zerstäuber (1000) mit der ersten Schnittstelle (710) verbunden ist, die zweite Schnittstelle (720) dazu ausgestaltet ist, Nebel zur Verwendung durch einen Benutzer abzugeben, und die dritte Schnittstelle (730) dazu ausgestaltet ist, Gas in das Dreiweg-Rohr (700) eintreten zu lassen; und wobei die Datenleitung (600) ein Eingangsende (610) und zumindest ein Ausgangsende (620) umfasst, das Eingangsende (610) mit dem Host (500) verbunden ist und das zumindest eine Ausgangsende (620) mit dem Zerstäuber (1000) verbunden ist.

8. Zerstäubungsvorrichtung nach Anspruch 7, wobei die Zerstäubungsvorrichtung ferner eine Gaskanalanordnung (800) umfasst, die Datenleitung (600) zwei Ausgangsenden (620) umfasst, eines der Ausgangsenden (620) mit dem Zerstäuber (1000) verbunden ist und das andere der Ausgangsenden (620) mit der Gaskanalanordnung (800) verbunden ist, die Gaskanalanordnung (800) einen Rohrkörper (810) und einen Durchflusssensor umfasst, der Durchflusssensor in dem Rohrkörper (810) angeordnet und dazu ausgestaltet ist, einen Gasdurchfluss zu messen, und ein Ende des Rohrkörpers (810) mit der dritten Schnittstelle (730) in Kommunikation steht und das andere Ende des Rohrkörpers (810) dazu ausgestaltet ist, Gas in den Rohrkörper (810) eintreten zu lassen.

9. Zerstäubungsvorrichtung nach Anspruch 7 oder 8, wobei ein zwischen einer Achse der ersten Schnittstelle (710) und einer Achse der zweiten Schnittstelle (720) eingeschlossener Winkel α ist und 90°<α<180° gilt.

## Revendications

1. Atomiseur (1000), comprenant :
un ensemble d'atomisation (100) comprenant une plaque d'atomisation (110) et conçu pour atomiser un liquide d'atomisation ; et
une coupelle d'atomisation (200) comprenant un corps de coupelle (210), un interface d'alimentation électrique (230) et une embouchure de coupelle (220), dans lequel l'interface d'alimentation électrique (230) est conçue pour être connectée à un connecteur électrique externe, le corps de coupelle (210) et l'interface d'alimentation électrique (230) sont situés du même côté de l'embouchure de coupelle (220), le corps de coupelle (210) comprend une partie de stockage de liquide (211), la partie de stockage de liquide (211) est dotée d'une cavité de stockage de liquide (21111), la cavité de stockage de liquide (2111) est conçue pour stocker le liquide d'atomisation, la coupelle d'atomisation (200) est en outre dotée d'une cavité d'atomisation (240), l'ensemble d'atomisation (100) est agencé dans la cavité d'atomisation (240), l'embouchure de coupelle (220) est dotée d'une sortie de gaz (221), et la sortie de gaz (221) est en communication avec la cavité d'atomisation (240) et est conçue pour évacuer le brouillard généré dans la cavité d'atomisation (240), dans lequel le corps de coupelle (210) comprend en outre une partie de connexion (212), un côté de la partie de connexion (212) est connecté à la partie stockage de liquide (211), et l'autre côté de la partie de connexion (212) est connecté à l'embouchure de coupelle (220), de façon à définir la cavité d'atomisation (240) avec l'embouchure de coupelle (220) ; et
dans lequel une partie de canal d'écoulement (300) est en outre agencée entre l'ensemble d'atomisation (100) et la partie de stockage de liquide (211), la partie de canal d'écoulement (300) est dotée d'un canal d'écoulement (310) faisant communiquer la cavité d'atomisation (240) avec la cavité de stockage de liquide (2111), la partie de stockage de liquide (211) et l'interface d'alimentation électrique (230) sont réparties le long d'une direction radiale du canal d'écoulement (310), et la partie de stockage de liquide (211) est agencé de manière oblique en s'éloignant de l'interface d'alimentation électrique (230), de façon à éviter l'interface d'alimentation électrique (230) ;
dans lequel l'ensemble d'atomisation (100) comprend en outre une plaque d'adaptateur (120), la plaque d'adaptateur (120) est dotée d'un trou traversant (121), le trou traversant (121) est en communication avec le canal d'écoulement (310), la plaque d'atomisation (110) est située sur un côté de la plaque d'adaptateur (12) en s'éloignant de l'interface d'alimentation électrique (230) et connectée électriquement à la plaque d'adaptateur (120), et la plaque d'adaptateur (120) est conçue pour être électriquement connectée au connecteur électrique externe ;
dans lequel, le long de la direction radiale du canal d'écoulement (310), un coin (320) est formé sur une paroi latérale du canal d'écoulement (310) en s'éloignant de l'interface d'alimentation électrique (230), une longueur entre le coin (320) et la plaque d'adaptateur (120) est L, L ≤ 3 mm, un diamètre du canal d'écoulement (310) est D, D ≥ 7 mm, et une partie de la cavité de stockage de liquide (2111) est située sur un côté du coin (320) en s'éloignant de l'interface d'alimentation électrique (230) ;
dans lequel l'atomiseur (1000) comprend en outre un élément étanche à l'eau (400), l'élément étanche à l'eau (400) est agencé dans la cavité d'atomisation (240), au moins une partie de l'élément étanche à l'eau (400) est serrée entre la partie de connexion (212) et l'ensemble d'atomisation (100), et l'élément étanche à l'eau (400) et la partie de connexion (212) sont conjointement conçus pour former la partie de canal d'écoulement (300) ; dans lequel l'élément étanche à l'eau (400) a une structure élastique, l'élément étanche à l'eau (400) est doté d'un trou de montage,
et **caractérisé en ce qu'**une paroi de trou du trou de montage est dotée d'une rainure de serrage, et un bord externe de l'ensemble d'atomisation (100) est serré dans la rainure de serrage ; dans lequel la rainure de serrage est renfoncée le long d'une direction radiale du trou de montage, et s'étend le long d'une direction circonférentielle du trou de montage, de façon à former une rainure annulaire fermée.

2. Atomiseur (1000) selon la revendication 1, dans lequel la plaque d'adaptateur (120) est conçue pour venir en butée contre le connecteur externe, et la plaque d'atomisation (110) est connectée de manière amovible à la plaque d'adaptateur (120).

3. Atomiseur (1000) selon la revendication 1, dans lequel le long de la direction radiale du canal d'écoulement (310), une distance entre un centre du trou traversant (121) et le coin (320) est supérieure à un rayon du canal d'écoulement (310).

4. Atomiseur (1000) selon la revendication 1, dans lequel l'embouchure de coupelle (220) est en outre dotée d'une rainure, la rainure est dotée d'une ouverture, le corps de coupelle (100) est connecté à l'ouverture, de manière à définir la cavité de stockage de liquide (2111), et l'ensemble d'atomisation (100) est serré entre l'embouchure de coupelle (220) et le corps de coupelle (210) .

5. Atomiseur (1000) selon la revendication 1, dans lequel la coupelle d'atomisation (200) comprend en outre une interface d'expansion (260), l'interface d'expansion (260) est connectée au corps de coupelle (210), l'interface d'expansion (260) est dotée d'un trou de remplissage de liquide, le trou de remplissage de liquide est en communication avec la cavité de stockage de liquide (2111), et l'interface d'expansion (260) est conçue pour être connectée à un récipient de stockage de liquide externe.

6. Dispositif d'atomisation, comprenant l'atomiseur (1000) selon l'une quelconque des revendications 1 à 5.

7. Dispositif d'atomisation selon la revendication 6, dans lequel le dispositif d'atomisation comprend en outre un hôte (500), une ligne de données (600) et un tube à trois voies ;
le tube à trois voies (700) comprend une première interface (710), une deuxième interface (720) et une troisième interface (730), l'atomiseur (1000) est connecté à la première interface (710), la deuxième interface (720) est conçue pour évacuer le brouillard pour un utilisateur en vue de l'utilisation, et la troisième interface (730) est conçue pour permettre au gaz d'entrer dans le tube à trois voies (700) ; et
la ligne de données (600) comprend une extrémité d'entrée (610) et au moins une extrémité de sortie (620), l'extrémité d'entrée (610) est connectée à l'hôte (500), et la au moins une extrémité de sortie (620) est connectée à l'atomiseur (1000).

8. Dispositif d'atomisation selon la revendication 7, dans lequel le dispositif d'atomisation comprend en outre un ensemble de canal de gaz (800), la ligne de données (600) comprend deux extrémités de sortie (620), l'une des extrémités de sortie (620) est connectée à l'atomiseur (600) et l'autre des extrémités de sortie (620) est connectée à l'ensemble de canal de gaz (800), l'ensemble de canal de gaz (800) comprend un corps de tube (810) et un capteur de débit, le capteur de débit est agencé dans le corps de tube (810) et conçu pour détecter un écoulement de gaz, et une extrémité du corps de tube (810) est en communication avec la troisième interface (730) et l'autre extrémité du corps de tube (810) est conçue pour permettre au gaz d'entrer dans le corps de tube (810).

9. Dispositif d'atomisation selon la revendication 7 ou 8, dans lequel un angle inclus entre un axe de la première interface (710) et un axe de la deuxième interface (720) est α, et 90° < α < 180°.
